# FASCICULE DE BREVET EUROPEEN

(11) **EP 2 654 765 B1**
(45) Date de publication et mention de la délivrance du brevet: **13.03.2019**
(21) Numéro de dépôt: 11801745.8
(22) Date de dépôt: 22.12.2011
(51) Int. Cl.: A61K 36/31, A23L 33/105, A61K 8/97, A61Q 19/08, A61P 17/00, A61P 9/00, A61K 8/368

(54) **EXTRAIT DE PARTIES AERIENNES DE MACA RICHE EN POLYPHENOLS ET COMPOSITION LE COMPRENANT**
EXTRAKT DER OBERIRDISCHEN TEILE DES MACA REICH AN POLYPHENOLE UND ZUSAMMENSETZUNGEN DIE DIESER EXTRAKT ENTHALTEN
EXTRACT OF THE AERIAL PARTS OF MACA RICH IN POLYPHENOL AND COMPOSITION COMPRISING THE EXTRACT

(30) Priorité: 22.12.2010 FR 1061047
(43) Date de publication de la demande: 30.10.2013
(73) Titulaire: Laboratoires Expanscience, 92048 Paris La Défense Cedex (FR)
(72) Inventeur: SAUNOIS, Alex, 28100 Dreux (FR); BAUDOUIN, Caroline, 78120 Rambouillet (FR); LECLERE-BIENFAIT, Sophie, 28100 Dreux (FR); GARNIER, Sébastien, 06650 Le Rouret (FR); MSIKA, Philippe, 78000 Versailles (FR)
(74) Mandataire: Regimbeau
(86) Numéro de dépôt international: PCT/EP2011/073834
(87) Numéro de publication internationale: WO 2012/085226

(56) Documents cités:
- EP-A1- 1 714 635
- EP-A1- 1 743 934
- DE-A1-102008 021 586
- FR-A1- 2 885 052
- FR-A1- 2 945 943
- US-A1- 2008 020 067
- DATABASE WPI Week 200770 Thomson Scientific, London, GB; AN 2007-745524 XP002652270, & JP 2007 230987 A (KINOSU KK) 13 septembre 2007 (2007-09-13)
- DATABASE WPI Week 200927 Thomson Scientific, London, GB; AN 2009-G61332 XP002652271, & JP 2009 067763 A (TSUJIDO KAGAKU KK) 2 avril 2009 (2009-04-02)
- DATABASE WPI Week 200731 Thomson Scientific, London, GB; AN 2007-314957 XP002652272, & JP 2007 031371 A (SUNTORY LTD) 8 février 2007 (2007-02-08)
- DATABASE WPI Week 201076 Thomson Scientific, London, GB; AN 2010-N35183 XP002652273, & JP 2010 235533 A (FANCL CORP) 21 octobre 2010 (2010-10-21)
- TELLEZ M R ET AL: "Composition of the essential oil of Lepidium meyenii (Walp.)", PHYTOCHEMISTRY, PERGAMON PRESS, GB, vol. 61, no. 2, 1 septembre 2002 (2002-09-01), pages 149-155, XP004374211, ISSN: 0031-9422, DOI: DOI:10.1016/S0031-9422(02)00208-X

## Description

L'invention se rapporte à un extrait de parties aériennes de Maca (*Lepidium meyenii*), avantageusement un extrait de feuilles de Maca, riche en polyphénols, et plus particulièrement riche en flavonoïdes, ainsi qu'à une composition comprenant un tel extrait. La composition est avantageusement cosmétique, pharmaceutique, dermatologique ou nutraceutique.

L'invention a également pour objet un procédé d'extraction d'un extrait de parties aériennes de Maca riche en polyphénols et plus particulièrement en flavonoïdes, ainsi que l'extrait susceptible d'être obtenu par ledit procédé.

L'invention concerne également une telle composition ou un tel extrait pour son utilisation dans la prévention ou le traitement des troubles ou pathologies de la peau, des muqueuses ou des phanères, pour son utilisation dans la prévention ou le traitement des troubles vasculaires, des réactions ou pathologies inflammatoires, ou encore pour son utilisation dans la prévention ou le traitement des régulations du tissu adipeux ou encore dans la prévention ou le traitement des altérations du tissu dermique. L'invention concerne enfin un procédé de soin cosmétique de la peau, des phanères ou des muqueuses, en vue d'améliorer leur état ou leur aspect, comprenant l'administration d'une telle composition ou d'un tel extrait.

### La plante Maca (Lepidium meyenii)

### Aspects botaniques et agronomiques

La Maca, *Lepidium meyenii* Walp, de la famille des Brassicaceae (Cruciferae) est une petite plante herbacée de 12 à 20 cm de haut. Sa partie souterraine mesure de 2 à 5 cm. Elle comprend une racine pivot surmontée de la partie basse d'un hypocotyle élargi et charnu. A l'état sec, l'ensemble rappelle la forme d'un petit navet. Selon les cultivars, les hypocotyles présentent une grande diversité de couleur allant du pourpre au jaune clair.

Les feuilles pennatifides forment une rosette et se renouvellent depuis son centre. Les petites fleurs sont autogames. Le fruit est une petite silique (4 à 5 mm) à deux valves comprenant chacune une graine.

### Composants chimiques de la graine et parties aériennes

La littérature ne signale aucune donnée chimique concernant ces organes de la Maca. Notons que les graines de plusieurs espèces de Lepidium cultivées aux Etats-Unis contiennent des quantités significatives de lipides (16 à 31 %).

### Propriétés biologiques

Les travaux publiés relatifs aux propriétés pharmacologiques de la Maca sont très rares.

La réputation de l'effet du tubercule de la Maca sur la fertilité a été confirmée par une étude pharmacologique menée chez le rat. Cette étude a permis de mettre en évidence un accroissement de la fertilité probablement due à l'augmentation du développement des follicules de Graaf. Les articles généraux qui rapportent cette information se réfèrent à des données d'accès difficiles dont une thèse péruvienne soutenue en 1961.

### Utilisations

Le tubercule de la Maca ou encore les hypocotyles ou racines de la Maca qui poussent sous terre ont déjà été utilisés dans l'art antérieur.

En particulier, la valeur nutritive du tubercule de la Maca, proche de céréales classiquement utilisées en alimentation, en fait un aliment de choix d'intérêt majeur pour les populations de haut plateaux péruviens. Il est consommé frais (sous forme de boisson fermentée, cuit sous la cendre), cuit (à l'état de confiture ou gâteau) ou bien sec (mélangé et bouilli avec de l'eau ou du lait sous forme de porridge, purée).

Le tubercule de la Maca est employé depuis des centaines d'années en usage populaire à des fins médicinales pour augmenter la fertilité des animaux et des êtres humains. Ainsi, les conquistadores espagnols auraient fortement accru la faible reproduction de leur cheptel grâce à la Maca.

Les Kallawaya, guérisseurs itinérants des Andes, prescrivaient aux femmes stériles désirant être fécondées, le tubercule frais découpé en fines rondelles, en décoction, trois ou quatre jours après les dernières règles.

Le tubercule est aussi utilisé frais, bouilli et réduit en purée en cataplasme abdominal pour provoquer les règles.

De nos jours, la popularité de la Maca s'accroît en raison des propriétés stimulantes et aphrodisiaques qui lui sont attribuées. Le tubercule de la Maca est apparenté (abusivement en raison d'un marché potentiel prometteur) au ginseng (*Panax ginseng*), d'où son nom de ginseng péruvien. Le pouvoir aphrodisiaque du tubercule serait du à la présence de prostaglandines et de stérols.

Parmi les autres utilisations du tubercule, figure son intérêt en cas de troubles respiratoires (tuberculose), fatigue chronique, troubles de la mémoire, symptômes de la ménopause, en cures lors de crises rhumatismales, ou encore pour son effet laxatif. Cependant, aucune de ces indications n'a été objectivée par des recherches scientifiques.

Par ailleurs, les parties aériennes de Maca sont habituellement considérées comme des déchets, et n'ont donc jamais été utilisées ou valorisées à des fins cosmétiques, dermatologiques, pharmaceutiques ou nutraceutiques.

### Art antérieur

Le document JP 2007 230987 décrit un médicament comprenant un extrait de Maca *(Lepidium meyenii Walp*) comme ingrédient actif et ayant une action contre l'obésité et / ou une action hypocholestérolémiante.

Le document US 2008/020067 concerne des hydratants pour la peau, et en particulier un hydratant pour la peau qui contient des extraits de plantes du genre Cruciferae, en particulier des plantes de Maca.

Le document EP 1 714 635 concerne un procédé d'extraction efficace d'extrait de Maca à l'aide d'éthanol.

Le document EP 1 743 934 concerne une boisson alcoolisée contenant un extrait de Maca.

Le document JP 2009 067763 décrit un extrait de Maca *(Lepidium meyenii Walp*) en tant qu'agent pour prévenir la « gueule de bois ».

Le document JP 2007 031371 décrit un extrait de Maca *(Lepidium meyenii Walp*) en tant qu'agent pour prévenir les troubles du sommeil.

Le document JP 2010 235533 décrit un extrait de feuilles de Maca obtenu par extraction solide-liquide avec de l'éthanol.

Le document TELLEZ et al. décrit la composition d'une huile de Maca obtenue par distillation.

Le document DE 10 2008 021586 décrit une composition nutritionnelle pour la production d'une boisson énergétique sportif pouvant comprendre un extrait de Maca en tant que composant vasoactif.

Le document FR 2 885 052 décrit l'utilisation d'un extrait de Maca (Lepidium meyenii) pour la préparation d'un médicament dermatologique à usage topique pour prévenir et traiter la déficience de la microcirculation cutanée.

Le document FR 2 945 943 document décrit un extrait de plante riche en polyphénols pouvant être obtenu par extraction solide-liquide.

### DESCRIPTION DE L'INVENTION

Les inventeurs ont découvert que les extraits de parties aériennes de Maca (*Lepidium meyenii*), notamment les extraits de feuilles, tiges, fleurs, ou graines, et avantageusement des extraits de feuilles, présentent des propriétés cosmétiques, dermatologiques ou pharmaceutiques jamais décrites jusqu'à présent.

En particulier, c'est la première fois que des extraits de parties aériennes de Maca sont utilisés en tant que tels, pour leurs propriétés spécifiques, notamment en vue d'améliorer la fermeté ou la tonicité de la peau, ou encore comme agents anti-âge.

L'invention a pour objet un extrait de parties aériennes de Maca riche en polyphénols, contenant au moins 5% en poids de polyphénols, exprimé en équivalent d'acide gallique, par rapport au poids de l'extrait sec.

On entend par extrait de parties aériennes de Maca riche en polyphénols, un extrait obtenu par des procédés permettant de concentrer les polyphénols potentiellement présents dans les parties aériennes de Maca de manière à ce que cet extrait contienne au moins 5% en poids de polyphenols exprimé en équivalent d'acide gallique par rapport au poids total de l'extrait sec, typiquement par dosage de Folin-Ciocalteu.

Selon une variante avantageuse de l'invention, l'extrait contient, exprimé en équivalent d'acide gallique par rapport à l'extrait sec obtenu, de 5% à 10% en poids de polyphénols.

La teneur en extrait sec dans l'extrait selon l'invention varie de 0,01 à 90%, avantageusement de 0,5 à 50%, plus avantageusement de 0,5 à 15%, encore plus avantageusement de 0,5 à 5%, en poids par rapport au poids total de l'extrait.

De manière particulièrement avantageuse selon l'invention, les polyphénols sont des flavonoïdes, tels que des flavonols.

L'extrait selon la présente invention contient au moins 2% en poids de flavonoïdes, exprimé en équivalent rutine, par rapport au poids total de l'extrait sec, typiquement par dosage au chlorure d'aluminium.

Avantageusement, l'extrait de parties aériennes de Maca contient au moins entre 2 et 4 % en poids de flavonoïdes, exprimé en équivalent rutine, par rapport au poids total de l'extrait sec.

Typiquement, les flavonoïdes de l'extrait selon la présente invention sont des flavonols, avantageusement choisis dans le groupe constitué par la quercétine, le kaempférol, leurs dérivés tels que leurs dérivés glycosylés, et leurs mélanges.

Dans un mode de réalisation particulier de la présente invention, les flavonoïdes contiennent la quercétine et le kaempférol, leurs dérivés tels que leurs dérivés glycosylés, ou leurs mélanges avantageusement à une concentration d'au moins 30%, typiquement d'au moins 50%, en poids, en particulier d'au moins 70%, avantageusement au moins 80%, plus avantageusement au moins 90%, exprimé en équivalent rutine, par rapport au poids total des flavonoïdes.

Selon une variante avantageuse de l'invention, l'extrait contient en outre de 0% à 70%, avantageusement de 10 à 60%, plus avantageusement de 20 à 50%, plus avantageusement de 30 à 40%, en poids de sucres, les % étant exprimés en poids par rapport au poids total de l'extrait sec, typiquement par dosage HPLC.

L'extrait selon l'invention contient par ailleurs avantageusement 0 à 50% en poids, plus avantageusement 0 à 20% en poids, encore plus avantageusement 0 à 10% en poids de lipides, les % étant exprimés en poids par rapport au poids total de l'extrait sec.

L'extrait selon l'invention contient par ailleurs avantageusement 0 à 60% en poids, plus avantageusement 0,5 à 30% en poids, encore plus avantageusement 0,5 à 10%, en poids de protéines, les % étant exprimés en poids par rapport au poids total de l'extrait sec, typiquement par dosage de Bradford.

Dans le cadre de la présente invention, les parties aériennes de Maca sont choisies dans le groupe constitué par les feuilles, les tiges, les fleurs, les graines, ou leurs mélanges.

Les tubercules de Maca, les hypocotyles de Maca, et les racines de Maca, qui poussent sous terre, ne font donc pas partie des parties aériennes de Maca au sens de la présente invention.

De manière particulièrement avantageuse, les parties aériennes sont des feuilles de Maca.

Cet extrait est susceptible d'être obtenu par extraction solide-liquide des parties aériennes, fraîches ou sèches, de Maca dans un solvant choisi dans des mélanges binaires eau/glycérol et/ou eau/glycol, dans des proportions comprises entre 30 et 90% de glycérol et/ou de glycol dans l'eau.

Majoritairement, on utilise des mélanges binaires de solvant du type eau et un solvant choisi parmi le glycérol ou le propanediol.

Dans un mode de réalisation particulier, le solvant d'extraction est un mélange hydro-glycérolique, et/ou hydro-glycolique, tel qu'un mélange eau-propanediol, dans une proportion comprise entre 30 et 90% de glycérol, et/ou de glycol dans l'eau.

Plus particulièrement, dans le cadre de l'extraction solide-liquide des parties aériennes, fraîches ou sèches, de Maca, on introduit entre 0,1 et 50% en poids (en équivalent de matière sèche) de parties de plante souhaitées dans le solvant d'extraction, et préférentiellement entre 1 et 10% en poids, typiquement 5% en poids (les % étant exprimés en poids de la matière sèche par rapport au poids total mis en oeuvre). La partie sèche de Maca peut être les feuilles, les tiges, les fleurs, les graines, seules ou associées, et préférentiellement les feuilles.

En particulier, en présence de glycérol, on choisit une proportion comprise entre 0 et 100% de glycérol dans l'eau, préférentiellement entre 30 et 80%, et avantageusement 50% (les % sont exprimés en poids de glycérol par rapport au poids total eau+glycérol).

En particulier, en présence de glycol et plus particulièrement de propanediol, on choisit une proportion comprise entre 0 et 100% de propanediol dans l'eau, préférentiellement entre 10 et 80%, et avantageusement 80% (les % sont exprimés en poids de propanediol par rapport au poids total eau+propanediol).

La température d'extraction est avantageusement comprise entre 4°C et 100°C, et préférentiellement entre 10°C et 60°C, et plus particulièrement entre 15°C et 30°C.

La durée d'extraction varie avantageusement de 30 minutes à 4h, et préférentiellement de 30 minutes à 2h, et plus avantageusement elle est d'environ 1h.

A l'issue de l'extraction, la matière sèche résiduelle est avantageusement séparée de la phase liquide, par exemple par filtration, décantation ou centrifugation. La phase liquide ainsi obtenue peut être filtrée à l'aide de filtres de porosité adaptée afin d'obtenir une solution limpide.

Ces premières étapes de séparation peuvent être suivies d'étapes de purification, par exemple par ultrafiltration et/ou nanofiltration, permettant de concentrer les molécules d'intérêt potentielles au dépend d'autres.

L'extrait obtenu pourra se présenter sous forme liquide mais également pourra être séché selon les méthodes connues de l'Homme de l'art, l'atomisation ou la lyophilisation par exemple avec, ou sans support telle la maltodextrine.

La description a également pour objet un procédé de préparation d'un extrait de parties aériennes de Maca, avantageusement des feuilles de Maca, comprenant au moins une étape d'extraction solide-liquide des parties aériennes de Maca dans un mélange binaire d'eau avec un solvant choisi dans le groupe constitué par les glycérols, les glycols tels que le propanediol, et leurs mélanges.

Typiquement, le procédé comprend les étapes successives suivantes :
(a) dispersion et extraction dans un solvant adapté de parties aériennes de Maca et avantageusement des feuilles, le solvant étant avantageusement un mélange binaire eau/glycérol et/ou eau/glycol,
(b) centrifugation et/ou filtration de l'extrait obtenu suite à l'étape (a) ;
(c) le cas échéant, ultrafiltration et/ou diafiltration et/ou nanofiltration de l'extrait obtenu suite à l'étape (b); puis
(d) récupération de l'extrait de parties aériennes de Maca ; et
(e) séchage éventuel de l'extrait obtenu à l'étape (d) sur un support ou non.

Lors de l'étape (a), on utilise avantageusement les parties aériennes, en particulier les feuilles, dans les proportions suivantes : entre 0,1 et 50% de matière sèche de feuilles, et préférentiellement entre 5 et 20%, et typiquement 5%, les pourcentages étant exprimés en poids de la matière sèche par rapport au poids total mis en oeuvre (parties aériennes + solvant).

L'extraction de l'étape (a) se fait avantageusement sous agitation. Aucune enzyme n'est ajoutée.

Lors de l'étape (a), on utilise avantageusement les solvants suivants en mélange avec l'eau : glycérol ou propanediol, dans une proportion avantageusement comprise entre 30 et 90% de ces solvants dans l'eau et plus avantageusement entre 50 et 80% (les % sont exprimés en poids du solvant par rapport au poids total solvant+eau).

L'invention a également pour objet une composition comprenant en tant qu'actif un extrait de parties aériennes de Maca, éventuellement en association avec un excipient approprié.

La composition est avantageusement cosmétique, pharmaceutique, dermatologique ou nutraceutique.

Ladite composition est de préférence formulée pour être administrée par voie topique externe ou orale.

Selon une variante avantageuse de l'invention, la composition contient 0,001 à 10 %, typiquement 0,01 à 5 %, avantageusement entre 1 et 5%, en poids d'extrait, exprimé en pourcentage d'extrait sec.

Selon un autre aspect de l'invention, la composition comprend en outre au moins un autre composé actif en plus de l'extrait de parties aériennes de Maca.

Cet autre composé peut être choisi parmi tous les composés et leurs équivalents fonctionnels, énoncés ci-dessous.

Cet autre composé peut être en particulier choisi parmi des actifs classiquement utilisés en dermatologie, en pharmaceutique ou en cosmétique et connus de l'Homme de l'art, tels que les émollients, les actifs hydratants, les kératorégulateurs, les kératolytiques, les agents cicatrisant et/ou restructurant de la barrière cutanée, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques (pigmentaires ou ultrafins), les composés antifongiques, les conservateurs, les agents anti-bactériens, les pré et probiotiques, les antibiotiques, les immnumodulateurs.

Plus particulièrement, les agents cicatrisants et/ou restructurants de la barrière cutanée pouvant être utilisés en association sont avantageusement le panthénol (vitamine B5), l'arabinogalactane, l'oxyde de zinc, les céramides, le cholestérol, le squalane et les phospholipides.

Les agents sébo-régulateurs pouvant être utilisés en association sont avantageusement choisis dans le groupe constitué par les inhibiteurs de 5-alpha réductase. Le zinc (et les dérivés du zinc tel que ses sels gluconate, salicylate et acide pyroglutamique) et la spironolactone, présentent aussi une activité sébo-suppresseur. D'autres séborégulateurs d'origine lipidique agissant sur la qualité du sébum, comme l'acide linoléique présentent un intérêt.

L'agent anti-inflammatoire et/ou anti-irritant et/ou apaisant peut être l'arabinogalactane.

Parmi les agents hypopigmentants ou dépigmentants, on peut notamment citer la N-undecylenoyl-L-phénylalanine (Sepiwhite®).

Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché.

Les conservateurs pouvant être utilisés en association sont par exemple ceux généralement utilisés en cosmétique et en nutraceutique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Parmi les actifs recommandés en association avec l'extrait selon l'invention, on peut citer les extraits végétaux, en particulier :
- Les huiles végétales telles que les huiles de Soja et/ou l'huile de Colza, l'huile d'avocat (WO2004/012496, WO2004/012752, WO2004/016106, WO2007/057439), l'huile de Lupin, avantageusement l'huile de Lupin blanc doux (WO 98/47479), ou un mélange de ces huiles ;
- l'oléodistillat ou les concentrats d'huile végétale ou animale, notamment de tournesol, plus avantageusement des concentrats de Tournesol linoléiques, tels que l'huile de tournesol concentrée en insaponifiables (Soline®)(cf. la demande internationale WO 01/21150) commercialisée par les Laboratoires Expanscience, les huiles concentrées en insaponifiable du type huile d'avocat, de colza, de maïs ou de palme, utiles notamment pour leur activité hydratante et/ou émolliente, cicatrisante et/ou restructurante de la barrière cutanée, anti-inflammatoire et/ou anti-irritante et/ou apaisante ;
- les insaponifiables de végétaux ou d'huile végétale, avantageusement des furanes d'avocat (Avocadofurane®), pouvant être obtenus par le procédé décrit dans la demande internationale WO 01/21605, les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®), les insaponifiables de soja (tels qu'obtenus selon le procédé décrit dans la demande internationale WO01/51596), les insaponifiables stéroliques (typiquement des insaponifiables dont la teneur en stérols, en méthylstérols et en alcools triterpèniques est comprise entre 20 et 95 % en poids, de préférence 45-65 % en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;

- les peptides ou complexes d'acides aminés végétaux, en particulier les peptides d'avocat (tel que ceux décrits dans la demande internationale WO2005/105123), les peptides de lupin (tels que ceux obtenus selon le procédé décrit dans la demande WO2005/102259), les peptides de quinoa (tel que ceux décrits dans la demande internationale WO2008/080974), les peptides de Maca tel que ceux décrits dans la demande internationale WO2004/112742), les peptides de soja fermenté ou non, les peptides de riz (tel que ceux décrits dans la demande internationale WO 2008/009709), utiles notamment pour leur activité hydratante et/ou émolliente (avocat), kératorégulatrice (lupin, quinoa), cicatrisante et/ou restructurante de la barrière cutanée (maca, quinoa, soja), anti-inflammatoire et/ou anti-irritante et/ou apaisante (lupin, quinoa), antioxydante (avocat), anti-âge (lupin, maca), pigmentante (riz) ;
- les sucres de végétaux, en particulier les sucres d'avocat (tel que ceux décrits dans la demande WO2005/115421), utiles notamment pour leur activité kératorégulatrice, cicatrisante et/ou restructurante de la barrière cutanée, anti-inflammatoire et/ou anti-irritante et/ou apaisante ;
- le butyle avocadate (5 alpha Avocuta®), inhibiteur de la 5-alpha réductase (voir WO 01/52837 et WO 02/06205), typiquement régulateur de la sécrétion séborrhée se trouvant augmentée dans l'acné ou les pellicules ;
- les extraits riches en polyphénols, et plus particulièrement les extraits de fruits d'avocat (tel que celui décrit dans la demande FR 1 061 047) et un extrait de parties aériennes de Gynandropsis gynandra (FR 1061051);
- le lupéol (FR 2 822 821, FR 2 857 596) utile notamment pour favoriser la cicatrisation,
- un extrait total de lupin (tel que ceux décrits dans la demande internationale WO2005/102259), particulièrement adapté pour le traitement des irritations ;
- un beurre de Cupuaçu, particulièrement apprécié pour ses propriétés hydratantes ;
- un extrait de graines d'*Acacia macrostachya* (FR 0958525), de graines de *Schizandra sphenanthera* (FR 0955343 et FR 0955344), et de graines de *Vigna unguiculata* (FR 0958529).

Parmi les actifs recommandés en association avec l'extrait selon l'invention, on peut citer les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline, de préférence la 2-undécyl-4,4-diméthyl-1,3-oxazoline (OX-100 ou Cyclocéramide® ; WO2004050052, WO2004050079, et WO2004112741). Elles sont particulièrement utiles pour leur activité anti-inflammatoire et/ou anti-irritante et/ou apaisante, antioxydante, dépigmentant, immunomodulatrice.

Parmi les actifs recommandés en association avec l'extrait selon l'invention, on peut citer les inhibiteurs de 5-alpha réductase, tels que le butyle avocadate (5 alpha Avocuta®).

Toutes ces associations comprennent au moins un autre composé actif, en plus de l'extrait de parties aériennes de Maca, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale.

La composition selon l'invention est avantageusement formulée sous la forme de différentes préparations adaptées à une administration topique, plus particulièrement pour application sur la peau, et/ou les phanères et/ou les muqueuses.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Selon une deuxième variante, les différentes préparations sont adaptées à une administration orale ; l'extrait de parties aériennes de Maca pouvant entrer soit dans un complément alimentaire soit dans une composition nutraceutique. Le complément alimentaire peut se présenter sous forme de l'extrait de parties aériennes de Maca en tant que tel ou bien sous forme de gélules ou de capsules molles de gélatine ou végétales dans le cadre de la présente invention. Ledit complément alimentaire peut alors contenir de 10 à 100% en poids de parties aériennes de Maca.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal, comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable, ou un excipient cosmétiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

La composition comprenant un extrait de parties aériennes de Maca ayant les spécifications indiquées est particulièrement destinée à une utilisation cosmétique, pharmaceutique, dermatologique ou nutraceutique.

Dans le cadre d'une utilisation cosmétique, pharmaceutique, ou dermatologique, la composition sera avantageusement formulée sous la forme d'une préparation adaptée à une administration topique.

Dans le cadre d'une utilisation à visée nutraceutique ou cosmétique («cosmet-food »), la composition sera avantageusement formulée sous la forme d'une préparation adaptée à une administration orale.

L'invention a également pour objet l'utilisation d'un extrait de parties aériennes de Maca pour la fabrication d'une composition cosmétique, pharmaceutique, dermatologique ou d'une composition nutraceutique.

Avantageusement, la composition ou l'extrait selon la présente invention est utilisée dans la prévention et/ou le traitement des troubles ou pathologies de la peau et/ou des muqueuses et/ou des phanères.

De manière particulièrement avantageuse, l'extrait ou la composition selon l'invention est utilisé dans des applications cosmétiques, avantageusement par voie topique, notamment pour le soin ou l'hygiène de la peau et/ou des muqueuses et/ou des phanères tels que les cheveux, ou encore pour la prévention et/ou le traitement des troubles de la peau et/ou des muqueuses et/ou des phanères tels que les cheveux.

La composition ou l'extrait selon la présente invention peut également être avantageusement utilisée dans la prévention et/ou le traitement des troubles vasculaires.

La composition ou l'extrait selon l'invention est particulièrement utile pour le renforcement des vaisseaux sanguins, des parois et du tonus vasculaire et/ou pour agir sur la stimulation de la circulation sanguine, et/ou pour agir sur les cellules endothéliales, et ainsi contribuer à un effet drainant pour lutter contre les jambes lourdes, les cernes, la cellulite, le relâchement cutané.

Le renforcement du tonus vasculaire favorise la vasoconstriction et le maintien de l'homéostasie des parois vasculaires.

La composition ou l'extrait selon la présente invention peut également être avantageusement utilisée dans la prévention et/ou le traitement des altérations du tissu dermique.

On peut citer comme exemple d'altération du tissu dermique les déséquilibres de la matrice dermique tels les rides, les problèmes de cicatrisation, la fermeté ou l'élasticité de la peau.

Ainsi, la composition ou l'extrait selon l'invention est également avantageusement utilisée dans la prévention du vieillissement, en particulier du vieillissement intrinsèque ou encore du vieillissement photo-induit et la peau vergeturée.

La composition ou l'extrait selon la présente invention peut également être avantageusement utilisée dans la prévention et/ou le traitement des régulations du tissu adipeux, et plus particulièrement pour la lutte contre l'accumulation du tissu adipeux et la peau avec cellulite.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

On entend par troubles de la barrière de la peau, des phanères et/ou des muqueuses les troubles intervenant au niveau de la couche extérieure de l'épiderme.

On entend par troubles de l'homéostasie de la peau, des phanères et/ou des muqueuses les troubles résultant des processus de renouvellement et d'équilibre des cellules telles que le psoriasis, la dermite du siège, la dermatite atopique, la peau sèche (xérose), la peau déshydratée et la peau photosensibilisée.

Avantageusement, la composition ou l'extrait selon l'invention peut être utilisé(e) pour la prévention et/ou le traitement des réactions, troubles ou pathologies :
- de la peau, telles que l'acné, la rosacée ou érythrocouperose, le psoriasis, la dermite du siège, la dermatite atopique, l'eczéma, la dermatite de contact, la dermatite irritative, la dermatite allergique, la dermite séborrhéique (croûte de lait), la peau sensible, la peau réactive, la peau sèche (xérose), la peau déshydratée, la peau avec rougeur, l'érythème cutanée, la peau photosensibilisée, la peau pigmentée (mélasma, pigmentation post-inflammatoire...), la peau dépigmentée (vitiligo), les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutismes, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

L'invention concerne également un procédé de soin cosmétique de la peau et/ou des phanères et/ou des muqueuses, en vue d'améliorer leur état et/ou leur aspect, comprenant l'administration ou consistant à administrer une composition cosmétique ou un extrait selon la présente invention.

Dans un mode de réalisation du procédé cosmétique selon l'invention, la composition ou l'extrait selon l'invention est utilisé en vue d'améliorer la fermeté, l'élasticité ou la tonicité de la peau, ou de prévenir le manque de fermeté ou de tonicité ou d'élasticité de la peau, on encore pour synthétiser et/ou protéger la matrice extracellulaire, ou encore pour prévenir et/ou traiter les altérations du tissu dermique.

Dans un autre mode de réalisation du procédé cosmétique selon l'invention, la composition ou l'extrait selon l'invention est utilisé comme agent antivieillissement ou anti-âge de la peau, des phanères ou des muqueuses, en particulier contre le vieillissement intrinsèque ou extrinsèque, notamment comme agent photo-vieillissement ou agent anti-UV, ou encore comme agent pour lutter contre l'accumulation du tissu adipeux et la peau avec cellulite.

Dans un autre mode de réalisation du procédé cosmétique selon l'invention, la peau et/ou les phanères et/ou les muqueuses visées sont avantageusement celles qui présentent un trouble de la circulation et du tonus vasculaire.

### Exemple 1

Les tiges feuillées de Maca séchées et broyées sont mises en suspension sous agitation à 5% dans un mélange éthanol/eau 10/90 p/p pendant 1 h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Extrait sec : 1,8%
▪ Sucres totaux (HPLC) : 32% /sec
▪ Polyphénols totaux (Folin - Ciocalteu) : 5,7% / sec
▪ Flavonoïdes totaux (AlCl3) : 1,7%

Cet extrait présente une activité anti-radicalaire, anti-DPPH « in tubo », pour laquelle la concentration inhibitrice 50 (IC50) a pu être déterminée et est de 0,29 mg d'extrait sec, ce qui représente 20 µg de polyphénols dans le milieu réactionnel.

### Exemple 2

Les tiges feuillées de Maca séchées et broyées sont mises en suspension sous agitation à 5% dans un mélange glycérol/eau 50/50 p/p pendant 1 h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Extrait sec : 1,8%
▪ Sucres totaux (HPLC) : 37% /sec
▪ Polyphénols totaux (Folin - Ciocalteu) : 6,4% / sec
▪ Flavonoïdes totaux (AlCl3) : 2,2 %/sec

Cet extrait présente une activité anti-radicalaire, anti-DPPH « in tubo », pour laquelle la concentration inhibitrice 50 (IC50) a pu être déterminée et est de 0,26 mg d'extrait sec, ce qui représente 16,5 µg de polyphénols dans le milieu réactionnel.

### Exemple 3

Les tiges feuillées de Maca séchées et broyées sont mises en suspension sous agitation à 5% dans un mélange propanediol/eau 80/20 p/p pendant 1 h à température ambiante. La matière sèche résiduelle est séparée de la phase liquide soit par filtration, décantation ou centrifugation et la phase liquide ainsi obtenue peut être filtrée à l'aide de filtres de porosité adaptée afin d'obtenir une solution limpide. L'extrait obtenu présente les caractéristiques suivantes :
▪ Extrait sec : 1,1%
▪ Sucres totaux (HPLC) : 40% /sec
▪ Polyphénols totaux (Folin - Ciocalteu) : 7,6% / sec
▪ Flavonoïdes totaux (AlCl3) : 3,6%

Cet extrait présente une activité anti-radicalaire, anti-DPPH « in tubo », pour laquelle la concentration inhibitrice 50 (IC50) a pu être déterminée et est de 0,28 mg d'extrait sec, ce qui représente 22,5 µg de polyphénols dans le milieu réactionnel.

### Exemple 4 : Compositions pour application par voie topique

Les inventeurs présentent ci-dessous plusieurs compositions pour application par voie topique. Les extraits de parties aériennes de Maca (PA Maca) peuvent être incorporés à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous. Les pourcentages représentent le poids du produit par rapport au poids total de la composition.

**EAU NETTOYANTE PEAU SENSIBLE**

| **Nom commercial ou INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| **Extrait de feuilles de maca** | De 0,01 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

**EMULSION ANTI-AGE**

| **Nom commercial ou INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| **Extrait de feuilles de maca** | De 0,01 à 10 % |
| ISONONYL ISONONANOAT | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**EMULSION RESTRUCTURANTE**

| **Matière première / Nom commercial ou INCI** | **%** |
|---|---|
| HYDROGENATED POL YDECENE | De 5 à 20 % |
| LAURYLGLUCOSIDE-GLYSTEARATE | De 1 à 5 % |
| DICAPRYLYL CARBONATE | De 1 à 5 % |
| GLYCEROL | De 5 à 20 % |
| CARBOPOL | De 0 à 1 % |
| GOMME XANTHANE | De 0 à 1 % |
| ACIDE ASIATIQUE | De 0 à 1 % |
| VITAMINE B5 | De 0 à 5 % |
| **Extrait de feuilles de maca** | De 0,01 à 10 % |
| LESSIVE SOUDE | De 0 à 1 % |
| CONSERVATEURS | De 0 à 1 % |
| ACIDE CITRIQUE | De 0 à 1 % |
| EAU PURIFIÉE | QSP 100 % |

**LAIT pour PEAU SECHE, ATOPIQUE**

| **Matière première / Nom commercial ou INCI** | **%** |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5% |
| **Extrait de feuilles de maca** | De 0,1 à 10 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**SPRAY APAISANT**

| **Matière première / Nom commercial ou INCI** | **%** |
|---|---|
| EAU PURIFIÉE | QSP 100 % |
| TRILAURETH-4 PHOSPHATE | De 1 à 5 % |
| DICAPRYLYL CARBONATE | De 1 à 5 % |
| BUTYLENE GLYCOL | De 1 à 5 % |
| ERYTHRITYL ESTER | De 1 à 5 % |
| HUILE VASELINE FLUIDE | De 1 à 5 % |
| BEURRE DE KARITE | De 0 à 1 % |
| HUILE VEGETALE | De 0 à 1 % |
| CONSERVATEURS | De 0 à 1 % |
| LYCOPENE | De 0 à 5 % |
| **Extrait de feuilles de maca** | De 0,01 à 10 % |
| LESSIVE SOUDE | De 0 à 1 % |
| PARFUM | De 0 à 1 % |
| GOMME XANTHANE | De 0 à 1 % |
| CARBOPOL | De 0 à 1 % |
| SEQUESTRANT | De 0 à 1 % |
| ACIDE CITRIQUE | De 0 à 1 % |

**SHAMPOOING ANTIPELLICULAIRE**

| **Matière première / Nom commercial ou INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| **Extrait de feuilles de maca** | De 0,01 à 10 % |
| ZINC PYRITHIONE | De 0 à 1 % |
| AJUSTEUR pH | De 0 à 1 % |
| SEQUESTRANT | De 0 à 1 % |
| PARFUM | De 0 à 1 % |

### Exemple 5 : Compositions pour administration par voie orale

Les extraits de feuilles de maca sont intégrés à des compositions orales, dans des compositions permettant l'administration de 50 mg à 200 mg d'extrait de feuilles de maca par jour.

### 1/ Composition anti-vergetures sous forme de capsules molles

| | |
|---|---|
| **- Extrait de parties aériennes de maca** | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine QSP 1 capsule molle | |

Cette composition est administrée de 4 à 6 capsules de 500 mg par jour.

### 2/ Comprimés anti-chute cheveux

| | |
|---|---|
| **- Extrait de parties aériennes de maca** | 25 mg |
| - Extraits de céréales (blé, sarrasin, millet, épeautre) riche en acides aminés soufrés | 200 mg |
| - Vitamine C | QSP 50 % des AJR |
| - Glycosaminoglycanes issus de cartilages de poissons | 200 mg |
| - Glucidex IT 19 (agent de compression) | QSP |
| | 1 comprimé de 800 mg. |

Cette composition est administrée de 5 à 8 comprimés par jour.

### 3/ Exemples en stick poudre amincissant

| | |
|---|---|
| **- Extrait de parties aériennes de maca** | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - ascorbate de sodium | 0,3 mg |
| - maltodextrine | QSP 5 g. |

Cette composition est administrée 2 fois par jour.

### Exemple 6 : Activités biologiques

### 1. Renforcement des parois vasculaires

### a. Activité sur cellules endothéliales

L'effet de l'extrait de feuilles de maca sur des cellules endothéliales dermiques a été étudié par un screening en PCR array.
Les cellules endothéliales sont les cellules constitutives de la paroi vasculaire, elles jouent donc un rôle prépondérant pour le maintien de l'équilibre vasculaire et régulent les interactions entre le vaisseau sanguin et les tissus environnants (le derme).

### ✔ Matériel et méthodes :

Des cellules endothéliales microvasculaires humaines ont été traitées par l'extrait de feuilles de maca (MC) à 0,005% et 0,01% (p/v de matière active) pendant 24 heures. Après incubation, l'expression génique de différents marqueurs a été analysée par RT-PCR quantitative en temps réel à l'aide d'un PCR array.

### ✔ Résultats et conclusion :

Les résultats du screening sur cellules endothéliales ont permis de montrer que l'extrait de feuilles de maca (tableau 1) :
- stimule l'expression génique de molécules impliquées dans le renforcement des vaisseaux : angiopoietine 1 (rôle dans l'établissement et la stabilisation des vaisseaux), serpine 1 (anti-protéase), cadhérine 5, fibrilline 1, intégrine αV (molécules d'adhésion cellule/cellule ou cellule/matrice jouant un rôle dans la stabilisation des vaisseaux), α SMA ou α smooth muscle actin et troponine 1 (renforcement des vaisseaux / élasticité) ;
- stimule l'expression génique de molécules impliquées dans le tonus vasculaire : calmoduline (contrôle de la perméabilité vasculaire), endothéline 1 et ECE1 ou Endothelin Converting Enzyme 1 (vasoconstriction) ;
- stimule l'expression génique de molécules de défense : Heme Oxygénase 1 (rôle protecteur en empêchant l'hème libre de participer à des réactions pro-oxydantes) et thioredoxine (réparation des dommages oxydatifs aux protéines) ; et
- inhibe l'expression génique de molécules impliquées dans l'inflammation : CSF1 ou macrophage colony-stimulating factor 1 (contrôle de la production/différenciation/fonction des macrophages) et sélectine E (molécule d'adhésion, rôle dans le recrutement de cellules inflammatoires).

Ces effets sont en faveur d'une activité stimulatrice du tonus vasculaire et de renforcement des vaisseaux sanguins.

**Tableau 1 : Screening d'activité en PCR array sur cellules endothéliales**

| | **Expression génique** (Quantité relative en % par rapport aux cellules contrôles) | | |
|---|---|---|---|
| | Cellules contrôles | **MC 0,005%** | **MC 0,01%** |
| **Renforcement des vaisseaux** | | | |
| Angiopoietine 1 | 100 | **142** | **207** |
| Serpine 1 | 100 | **139** | **157** |
| Cadhérine 5 | 100 | **121** | **198** |
| Fibrilline 1 | 100 | **155** | **199** |
| Intégrine αV | 100 | **145** | **174** |
| α SMA | 100 | **119** | **147** |
| Troponine 1 | 100 | **116** | **201** |

| **Tonus vasculaire** | | | |
|---|---|---|---|
| Calmoduline | 100 | **121** | **151** |
| Endothéline 1 | 100 | **119** | **144** |
| ECE 1 | 100 | **117** | **162** |

| **Réponse au stress** | | | |
|---|---|---|---|
| Hème oxygénase 1 | 100 | **143** | **158** |
| Thiorédoxine | 100 | **131** | **140** |

| **Inflammation** | | | |
|---|---|---|---|
| CSF 1 | 100 | **57** | **106** |
| Sélectine E | 100 | **49** | **69** |

### 2. Protection et renforcement de la matrice dermique

### a. Activité sur des marqueurs de la matrice dermique

L'activité de l'extrait de feuilles de maca sur la matrice dermique a été recherchée dans des fibroblastes dermiques en analysant l'expression génique de différents marqueurs :
- le collagène de type I, composant majoritaire de la matrice extra-cellulaire dermique, responsable de la fermeté et de la résistance de la peau ;
- l'élastine, responsable de l'élasticité de la peau ;
- la MMP1 (matrix metalloprotease de type 1), responsable de la dégradation des collagènes de type I et III ;
- la dermatopontine qui joue un rôle dans l'assemblage des fibres de collagène, elle est importante pour le maintien de l'architecture de la matrice extra-cellulaire dermique et la flexibilité du tissu ;
- la décorine qui se lie aux fibres de collagène pour en empêcher le clivage par la MMP1 ;
- la fibronectine : glycoprotéine de haut poids moléculaire qui joue un rôle dans la structure de la matrice extracellulaire dermique ;
- la hyaluronane synthase 2 : enzyme impliquée dans la formation de l'acide hyaluronique, molécule hygroscopique importante pour l'hydratation et la densité de la peau.

### ✔ Matériel et méthode :

Des fibroblastes dermiques humains normaux ont été traités pendant 24 heures par l'extrait de feuilles de maca (MC) à 0,002% et 0,005% (p/v de matière active) ou par le TGFβ1 à 5 ng/ml (référence).
A la fin de l'incubation, l'expression génique de différents marqueurs de la matrice dermique a été analysée par RT-PCR en temps réel.

Les résultats ont été analaysés statistiquement par une ANOVA à un facteur suivie d'un test de Dunnett.

### ✔ Résultats et conclusion :

Comme le montre le tableau 2, l'extrait de feuilles de maca a significativement augmenté l'expression génique du collagène I, de l'élastine, de la dermatopontine, de la décorine, de la fibronectine et de la hyaluronane synthase 2.
D'autre part, l'extrait de feuilles de maca a significativement inhibé l'expression génique de la MMP1.

Ces résultats sont en faveur d'un renforcement de la matrice extra-cellulaire dermique.

### Tableau 2 : Expression génique de marqueurs de la matrice dermique dans des fibroblastes (Quantité Relative)

| | Contrôle | TGFβ1 | | **MC 0,002%** | | **MC 0,005%** | |
|---|---|---|---|---|---|---|---|
| **Collagène I** | 1,00 | 1,40 | +40% | **1,59** | +59% (p<0,001) | **1,23** | +23% |
| **Elastine** | 1,00 | 1,57 | +57% | **1,66** | +66% (p<0,05) | **1,25** | +25% |
| **MMP1** | 1,00 | 0,22 | -78% (p<0,001) | **0,58** | -42% (p<0,001) | **0,72** | -28% (p<0,05) |
| **Dermatopontine** | 1,00 | 1,34 | +34% (p<0,05) | **1,37** | +37% (p<0,01) | **1,28** | +28% (p<0,05) |
| **Décorine** | 1,00 | *nd* | *nd* | **1,35** | +35% (p<0,05) | **1,15** | +15% |
| **Fibronectine** | 1,00 | 2,41 | +141% (p<0,001) | **1,53** | +53% (p<0,01) | **1,42** | +42% (p<0,05) |
| **Hyaluronane synthase 2** | 1,00 | *nd* | *nd* | **1,40** | +40% (p<0,01) | **1,20** | +20% |

| | | | | | | | |
|---|---|---|---|---|---|---|---|
| nd : non déterminé | | | | | | | |

### b. Activité sur un marqueur de la matrice dermique dans des fibroblastes vieillis

L'effet de l'extrait de feuilles de maca a été recherché dans des fibroblastes dermiques prématurément vieillis par traitement à l'H₂O₂, par analyse de l'expression génique du biglycane.
Le biglycane est un petit protéoglycane qui influence la fibrillogénèse du collagène.

### ✔ Matériel et méthode :

Des fibroblastes dermiques humains normaux ont été prématurément vieillis par de l'H₂O₂ à 600 µM.

Les fibroblastes ont ensuite été incubés pendant 72 heures en présence de l'extrait de feuilles de maca (MC) à 0,005% et 0,01% (p/v de matière active) ou de TGFβ à 10 ng/ml (référence).

Après incubation, l'expression génique du biglycane a été analysée par RT-PCR quantitative en temps réel.

### ✔ Résultats et conclusion :

L'induction de la sénescence des fibroblastes par le traitement à l'H₂O₂ a inhibé l'expression du biglycane, l'extrait de feuilles de maca a permis de restaurer cette expression (tableau 3).

### L'extrait de feuilles de maca protège la matrice dermique dans des conditions de sénescence.

**Tableau 3 : Expression génique du biglycane dans des fibroblastes dermiques vieillis par H₂O₂**

| **Biglycane** | | | |
|---|---|---|---|
| (Quantité relative) | | | |
| Fibroblastes contrôles | Fibroblastes vieillis (H₂O₂) | **MC 0,005%** | **MC 0,01%** |
| 123 | 100 | **112** | **142** |

### c. Inhibition de la production d'élastase

Sous l'influence de différents stimuli (inflammation, irradiation UV...), les polynucléaires neutrophiles humains libèrent des espèces oxygénées et des enzymes telles que l'élastase.
Un excès d'élastase peut dégrader les protéines matricielles de la peau, telles que l'élastine, les protéoglycanes, le collagène, la décorine ...

L'effet de l'extrait de feuilles de maca a été évalué sur la libération d'élastase par des neutrophiles humains.

### ✔ Matériel et méthode :

Des neutrophiles humains ont été traités pendant 15 minutes par l'extrait de feuilles de maca (MC) à 0,005 et 0,01% (p/v de matière active) ou par l'acide boswellique à 10 µg/ml (référence) puis stimulés par le calcium ionophore A23187à 1 µM, pendant 10 minutes.

La libération d'élastase a été évaluée par mesure de l'activité élastase par une méthode spectrophotométrique.

Les résultats ont été analysés statistiquement par un test t de Student.

### ✔ Résultats et conclusion :

L'extrait de feuilles de maca a significativement inhibé la libération d'élastase par des neutrophiles (tableau 4).

Ces résultats montrent un effet protecteur de la matrice dermique.

**Tableau 4 : Libération d'élastase par des neutrophiles**

| | **Elastase** (libération nette ; %) | Inhibition | |
|---|---|---|---|
| Calcium ionophore | 100 ± 1,70 | | |
| Acide boswellique 10 µg/ml | 50,90 ± 7,49 | **-49%** | **p<0,001** |
| **MC 0,005%** | **81,40 ± 0,90** | **-19%** | **p<0,001** |
| **MC 0,01%** | **60,24 ± 5,96** | **-40%** | **p<0,001** |

### 3. Défenses anti-inflammatoires et anti-oxydantes

### a. Protection contre la peroxydation lipidique

### ✔ Matériel et méthodes :

Des cellules de lignée Jurkat ont été pré-incubées pendant 45 minutes en présence de l'extrait de feuilles de maca (MC) à 0,005% et 0,01% (p/v de matière active) ou du BHT à 100 µM (référence) et en présence de la sonde fluorescente C11-fluor, spécifique de la peroxydation des lipides.

Les cellules ont ensuite été irradiées par des UVA+B puis incubées pendant 30 minutes en présence de MC ou du BHT.

A la fin de l'incubation, la quantité de peroxydes lipidiques a été évaluée par analyse en cytométrie en flux de l'intensité de fluorescence (inversement proportionnelle à l'oxydation).

Les résultats ont été analysés statistiquement par un test t de Student.

### - Résultats et conclusion :

L'extrait de feuilles de maca a significativement protégé les cellules contre la peroxydation lipidique induite par l'irradiation aux UV (tableau 5).

**Tableau 5 : Peroxydation des lipides induite par UV**

| | **Peroxydes lipidiques** (% du témoin irradié) | Protection (%) | |
|---|---|---|---|
| Cellules irradiées (UV) | 100 | | |
| BHT 100 µM | 64 | 49% | p< 0,01 |
| **MC 0,005%** | **62** | **52%** | **p<0,01** |
| **MC 0,01%** | **59** | **56%** | **p<0,01** |

### b. Inhibition de l'inflammation induite par le PMA

### ✔ Matériel et méthodes :

Des kératinocytes humains (lignée NCTC-2544) ont été pré-incubés ou non (contrôle) par l'extrait de feuilles de maca (MC) à 0,005% et 0,01% (p/v de matière active) ou les molécules de référence anti-inflammatoires (dexaméthasone à 10⁻⁷M ; indométhacine à 10⁻⁶M) pendant 24 heures. Les cellules ont ensuite été traitées par le PMA à 0,1 µg/ml (Phorbol Myristate Acetate) pendant 24 heures, toujours en présence de MC ou des références.
A l'issue du traitement, les quantités d'IL8 (interleukine 8) et de PGE2 (prostaglandine E2) sécrétées ont été mesurées par ELISA dans les surnageants de culture.

Les résultats ont été analysés statistiquement par un test t de Student.

### ✔ Résultats et conclusion :

L'extrait de feuilles de maca a fortement et significativement inhibé la production des médiateurs inflammatoires IL8 et PGE2 stimulée par le PMA dans des kératinocytes (tableau 6).

Ces résultats montrent l'activité anti-inflammatoire de l'extrait de feuilles de maca.

**Tableau 6 : Production d'IL8 et de PGE2 par des kératinocytes**

| | **IL8** (ng/ml) | Inhibition | |
|---|---|---|---|
| Cellules contrôles | 0,1 ± 0,0 | | |
| PMA 0,1 µg/ml | 50,1 ± 1,8 | | |
| Dexaméthasone 10-7M | 7,4 ± 0,8 | 85% | p<0,001 |
| **MC 0,005%** | **24,7 ± 0,8** | **51%** | **p<0,001** |
| **MC 0,01%** | **19,3 ± 0,8** | **62%** | **p<0,001** |

| | **PGE2** (ng/ml) | Inhibition | |
|---|---|---|---|
| Cellules contrôles | 0,039 ± 0,0 | | |
| PMA 0,1 µg/ml | 138,4 ± 10,6 | | |
| Indométhacine 10-6M | 0,039 ± 0,0 | 100% | p<0,001 |
| **MC 0,005%** | **21,8 ± 1,8** | **84%** | p<0,001 |
| **MC 0,01%** | **9,8 ± 0,0** | **93%** | p<0,001 |

### c. Inhibition de l'inflammation induite par P. Acnes

L'effet protecteur de l'extrait de feuilles de maca vis-à-vis d'une inflammation induite par le *propionibacterium acnes* a été étudié sur des kératinocytes colonisés par cette bactérie.

### ✔ Matériel et méthodes :

Des kératinocytes humains (lignée HaCaT) ont été pré-incubés pendant 48h en présence de l'extrait de feuilles de maca (MC) à 0,008% et 0,031% (p/v de matière active) ou de l'inhibiteur de référence : nicotinamide.
Les kératinocytes ont alors été stimulés par incubation pendant 18 heures avec une suspension bactérienne de *P. acnes* (souche ATCC6919).
A la fin de l'incubation, la quantité d'IL8 produite par les kératinocytes a été mesurée dans les surnageants de culture par une technique ELISA.

Les résultats ont été analysés statistiquement par un test t de Student : ns p>0,05 (non significatif) ; *p<0,05 ; **p<0,01 ; ***p<0,001.

### ✔ Résultats et conclusion :

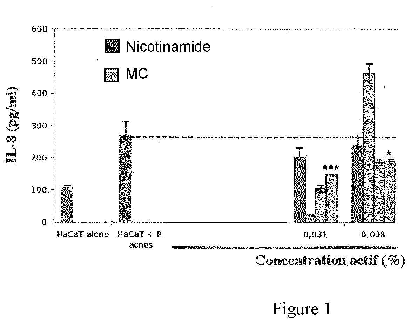

La figure 1 représente la production d'IL8, en pg/ml, par des kératinocytes stimulés par *P.acnes,* avec un temps de pré-incubation de 18h, en fonction de la concentration d'actif en %.
L'extrait de feuilles de maca a significativement inhibé la production d'IL8 induite par *P. acnes* sur des kératinocytes (figure 1).
L'extrait de feuilles de maca module donc l'inflammation induite par *P*. *acnes.*

### 4. Inhibition de la lipogénèse dans des adipocytes

### ✔ Matériel et méthodes :

Des adipocytes humains normaux ont été incubés pendant 1 heure en présence de l'extrait de feuilles de maca (MC) à 0,005% et 0,01% (p/v de matière active) ou de la référence (cérulénine à 20 µM). Après incubation, le marqueur radioactif [¹⁴C]-acétate a été ajouté et les échantillons ont été incubés la nuit.
A la fin de l'incubation, les lipides ont été extraits et la radioactivité incorporée (correspondant à la lipogénèse) a été mesurée par scintillation liquide.

Les résultats ont été analysés statistiquement par un test t de Student.

### - Résultats et conclusion :

L'extrait de feuilles de maca a significativement inhibé la néosynthèse des lipides par des adipocytes (tableau 7).
Ainsi, cet extrait présente donc un effet amincissant.

**Tableau 7 : Evaluation de la lipogénèse dans des adipocytes**

| | **Incorporation d'acétate** (cpm) | Inhibition | |
|---|---|---|---|
| Cellules contrôles | 32895 ± 1358 | | |
| Référence (Cérulénine) | 14934 ± 671 | 55% | p<0,001 |
| **MC 0,005%** | **27331 ± 700** | **17%** | **p<0,05** |
| **MC 0,01%** | **23664 ± 960** | **28%** | **p<0,01** |

## Revendications

1. Extrait de parties aériennes de Maca contenant au moins 5% en poids de polyphénols, exprimé en équivalent d'acide gallique, par rapport au poids total de l'extrait sec, lesdits polyphénols étant des flavonoïdes à une teneur supérieure à 2% en flavonoïdes, exprimé en équivalent de rutine par rapport au poids total de l'extrait sec, ledit extrait étant susceptible d'être obtenu par extraction solide-liquide des parties aériennes de Maca dans des mélanges binaires eau/glycérol et/ou eau/glycol, dans une proportion comprise entre 30 et 90% de glycérol et/ou de glycol dans l'eau.

2. Extrait selon la revendication 1, **caractérisé en ce que** les flavonoïdes contiennent la quercétine, le kaempférol, leurs dérivés, ou leurs mélanges, avantageusement à une concentration d'au moins 30%, typiquement d'au moins 50%, en poids, exprimé en équivalent d'acide gallique, par rapport au poids total des flavonoïdes.

3. Extrait selon les revendications 1 ou 2, **caractérisé en ce que** les parties aériennes sont des feuilles de Maca.

4. Composition comprenant en tant qu'actif un extrait selon l'une quelconque des revendications précédentes et avantageusement un excipient approprié, **caractérisée en ce qu'**il s'agit d'une composition cosmétique, pharmaceutique, dermatologique ou nutraceutique.

5. Composition selon la revendication 4, comprenant en outre au moins un autre composé actif en plus de l'extrait de parties aériennes de Maca, en particulier choisi dans le groupe constitué par les émollients, les actifs hydratants, les kératorégulateurs, les kératolytiques, les agents cicatrisant et/ou restructurant de la barrière cutanée, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, et les immunomodulateurs.

6. Composition selon la revendication 5, **caractérisée en ce que** l'autre actif est choisi parmi :
• les agents cicatrisant et/ou restructurant de la barrière cutanée, de préférence le panthénol, l'arabinogalactane et l'oxyde de zinc,
• les agents sébo-régulateurs, de préférence choisis parmi les inhibiteurs de 5-alpha réductase, les dérivés de zinc, la spironolactone, et l'acide linoléique,
• les agents anti-inflammatoires et/ou anti-irritants et/ou apaisants, de préférence l'arabinogalactane,
• les agents hypopigmentants ou dépigmentants, de préférence la N-undecylenoyl- L-phénylalanine.
• les filtres et écrans solaires minéraux ou organiques, de préférence les filtres et écrans solaires UVB et/ou UVA, et
• les conservateurs de préférence choisis parmi le capryloyl glycine, le glycéryl caprylate, l'hexanediol, le sodium levulinate, les dérivés de zinc et de cuivre.

7. Composition selon l'une quelconque des revendications 5 à 6, comprenant en outre au moins un autre actif choisi dans le groupe constitué parmi :
• les huiles végétales, de préférence l'huile de soja, l'huile de colza, l'huile d'avocat, l'huile de lupin et avantageusement l'huile de lupin blanc doux, ou un mélange de ces huiles,
• les oléodistillats ou les concentrats d'huile végétale ou animale, de préférence de tournesol, d'avocat, de colza, de maïs et de palme et avantageusement concentrés en insaponifiables,
• les insaponifiables de végétaux ou d'huile végétale, de préférence les insaponifiables d'avocats, les insaponifiables de soja ou leurs mélanges, avantageusement des furanes d'avocat, et en particulier un mélange d'insaponifiables d'Avocat furaniques et d'insaponifiables de Soja dans un rapport respectif d'environ 1/3-2/3, les insaponifiables stéroliques, les phytostérols, les esters de stérols et les dérivés vitaminiques,
• les peptides ou complexes d'acides aminés végétaux, de préférence les peptides d'avocat, les peptides de lupin, les peptides de quinoa, les peptides de Maca, les peptides de soja fermentés ou non, les peptides de riz,
• les sucres de végétaux, de préférence les sucres d'avocat,
• le butyle avocadate,
• les extraits riches en polyphénols, de préférence les extraits d'avocat et les extraits de parties aériennes de *Gynandropis gynandra*,
• le lupéol,
• un extrait total de lupin,
• un beurre de Cupuaçu,
• un extrait de graines *d'Acacia macrostachya*, un extrait de graines de *Schizandra sphnanthera* et un extrait de graines de *Vigna unguiculata*,
• les oxazolines, de préférence la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline et la 2-undécyl-4,4-diméthyl-1,3-oxazoline, et
leurs mélanges.

8. Composition selon l'une quelconque des revendications 4 à 7, **caractérisée en ce qu'**elle est formulée pour être administrée par voie topique ou orale.

9. Composition selon l'une quelconque des revendications 4 à 8 ou extrait tel qu'il est défini dans l'une quelconque des revendications 1 à 3 pour son utilisation dans la prévention et/ou le traitement des troubles ou pathologies de la peau et/ou des muqueuses et/ou des phanères.

10. Composition selon l'une quelconque des revendications 4 à 8 ou extrait tel qu'il est défini dans l'une quelconque des revendications 1 à 3 pour son utilisation dans la prévention et/ou le traitement des troubles vasculaires, et plus particulièrement visant à renforcer les parois et le tonus vasculaires.

11. Composition selon l'une quelconque des revendications 4 à 8 ou extrait tel qu'il est défini dans l'une quelconque des revendications 1 à 3 pour son utilisation dans la prévention et/ou au traitement des réactions ou pathologies allergiques, inflammatoires, irritatives ou des troubles de la barrière ou de l'homéostasie de la peau, des phanères et/ou des muqueuses immature(s), normale(s) ou mature(s)/âgée(s).

12. Composition selon l'une quelconque des revendications 4 à 8 ou extrait tel qu'il est définit dans l'une quelconque des revendications 1 à 3 pour son utilisation dans la prévention et/ou le traitement des altérations du tissu adipeux, et plus particulièrement le traitement des régulations du tissu adipeux, ou pour prévenir et/ou traiter les troubles de la circulation sanguine et renforcer le tonus vasculaire.

13. Procédé de soin cosmétique de la peau et/ou des phanères et/ou des muqueuses, en vue d'améliorer leur état et/ou leur aspect, avantageusement en vue d'améliorer la fermeté, l'élasticité ou la tonicité de la peau, et pour lutter contre l'accumulation du tissu adipeux et la peau avec cellulite, ou encore pour prévenir et/ou traiter les altérations du tissu dermique, ou encore pour prévenir et/ou traiter le vieillissement, comprenant l'administration d'une composition cosmétique telle que définie selon l'une quelconque des revendications 4 à 8 ou un extrait tel que défini selon l'une quelconque des revendications 1 à 3.

## Patentansprüche

1. Extrakt aus oberirdischen Maca-Teilen, enthaltend mindestens 5 Gew.-% an Polyphenolen, ausgedrückt in Gallussäureäquivalent, in Bezug auf das Gesamtgewicht des trockenen Extrakts, wobei die Polyphenole Flavonoide mit einem Gehalt von über 2 % an Flavonoiden, ausgedrückt in Rutinäquivalent in Bezug auf das Gesamtgewicht des trockenen Extrakts, sind, wobei der Extrakt durch Fest-Flüssig-Extraktion der oberirdischen Maca-Teile in binären Wasser-Glycerol- und/oder Wasser-Glycol-Gemischen in einem Verhältnis zwischen 30 und 90 % Glycerol und/oder Glycol in Wasser erhätlich ist.

2. Extrakt nach Anspruch 1, **dadurch gekennzeichnet, dass** die Flavonoide das Quercetin, das Kaempferol, ihre Derivate oder ihre Gemische in vorteilhafter Weise in einer Konzentration von mindestens 30 Gew.-%, in typischer Weise von mindestens 50 Gew.-%, ausgedrückt in Gallussäureäquivalent in Bezug auf das Gesamtgewicht der Flavonoide, enthalten.

3. Extrakt nach den Ansprüchen 1 oder 2, **dadurch gekennzeichnet, dass** die oberirdischen Teile Maca-Blätter sind.

4. Zusammensetzung, umfassend als Wirkstoff einen Extrakt nach einem der vorangehenden Ansprüche und in vorteilhafter Weise einen geeigneten Hilfsstoff, **dadurch gekennzeichnet, dass** es sich um eine kosmetische, pharmazeutische, dermatologische oder nutrazeutische Zusammensetzung handelt.

5. Zusammensetzung nach Anspruch 4, ferner umfassend mindestens eine andere aktive Verbindung neben dem Extrakt aus oberirdischen Maca-Teilen, die insbesondere aus der Gruppe ausgewählt ist, die aus den Weichmachern, den feuchtigkeitsspendenden Wirkstoffen, den Keratinregulierern, den keratolytischen Mitteln, den heilenden und/oder die Hautbarriere restrukturierenden Mitteln, den sebumregulierenden Mitteln, den gegen Reizungen und/oder Entzündungen wirkenden und/oder besänftigenden Mitteln, den Antioxidantien, den Anti-Age-Mitteln, den depigmentierenden oder hypopigmentierenden Mitteln, den pigmentierenden Mitteln, den lipolytischen oder die Lipogenese hemmenden Mitteln oder auch den Anti-Cellulite- oder figurformenden Mitteln, den mineralischen oder organischen Sonnenfiltern und - schirmen, den fungiziden Verbindungen, den Konservierungsmitteln, den antibakteriellen Mitteln, den Prä- und Probiotika, den Antibiotika und den Immunmodulatoren besteht.

6. Zusammensetzung nach Anspruch 5, **dadurch gekennzeichnet, dass** der andere Wirkstoff ausgewählt ist aus:
• den heilenden und/oder die Hautbarriere restrukturierenden Mitteln, vorzugsweise dem Panthenol, dem Arabinogalactan und dem Zinkoxid,
• den sebumregulierenden Mitteln, die vorzugsweise aus den 5-Alpha-Reduktasehemmern, den Zinkderivaten, dem Spironolacton und der Linolsäure ausgewählt sind,
• den gegen Reizungen und/oder Entzündungen wirkenden und/oder besänftigenden Mitteln, vorzugsweise dem Arabinogalactan,
• den depigmentierenden oder hypopigmentierenden Mitteln, vorzugsweise dem N-undecylenoyl-L-phenylalanin,
• den mineralischen oder organischen Sonnenfiltern und -schirmen, vorzugsweise den UVB- und/oder UVA-Sonnenfiltern und -schirmen, und
• den Konservierungsmitteln, vorzugsweise ausgewählt aus dem Capryloylglycin, dem Glycerylcaprylat, dem Hexandiol, dem Natriumlevulinat, den Derivaten von Zink und von Kupfer.

7. Zusammensetzung nach einem der Ansprüche 5 bis 6, ferner umfassend mindestens einen anderen Wirkstoff, der aus der Gruppe ausgewählt ist, die besteht aus:
• den pflanzlichen Ölen, vorzugsweise dem Sojaöl, dem Rapsöl, dem Avocadoöl, dem Lupinenöl und in vorteilhafter Weise dem Öl der süßen weißen Lupine oder einem Gemisch dieser Öle,
• den Öldestillaten oder den Konzentraten aus pflanzlichem oder tierischem Öl, vorzugsweise von Sonnenblume, Avocado, Raps, Mais und Palme und in vorteilhafter Weise konzentriert an unverseifbaren Fraktionen,
• den unverseifbaren Fraktionen von Pflanzen oder pflanzlichen Ölen, vorzugsweise den unverseifbaren Fraktionen von Avocado, den unverseifbaren Fraktionen von Soja oder deren Gemischen, in vorteilhafter Weise der Avocadofurane, und insbesondere einem Gemisch aus unverseifbaren Fraktionen von Avocadofuranen und unverseifbaren Fraktionen von Soja in einem jeweiligen Verhältnis von zirka 1/3-2/3, den unverseifbaren Sterolfraktionen, den Phytosterolen, den Sterolestern und den Vitaminderivaten,
• den Peptiden oder Komplexen pflanzlicher Aminosäuren, vorzugsweise den Avocadopeptiden, den Lupinenpeptiden, den Quinoapeptiden, den Macapeptiden, den fermentierten oder nicht fermentierten Sojapeptiden, den Reispeptiden,
• den pflanzlichen Zuckern, vorzugsweise den Avocadozuckern,
• dem Butylavocadat,
• den an Polyphenolen reichen Extrakten, vorzugsweise den Avocadoextrakten und den Extrakten aus den oberirdischen Teilen von *Gynandropis gynandra,*
• dem Lupeol,
• einem Lupinen-Totalextrakt,
• einer Cupuacu-Butter,
• einem Extrakt aus Samen von *Acacia macrostachya,* einem Extrakt aus Samen von *Schizandra sphnanthera* und einem Extrakt aus Samen von *Vigna unguiculata,*
• den Oxazolinen, vorzugsweise dem 2-Undecyl-4-hydroxymethyl-4-methyl-1,3-oxazolin, dem 2-Undecyl-4,4-dimethyl-1,3-oxazolin, dem (E)-4,4-Dimethyl-2-heptadec-8-enyl-1,3-oxazolin, dem 4-Hydroxymethyl-4-methyl-2-heptadecyl-1,3-oxazolin, dem (E)-4-Hydroxymethyl-4-methyl-2-heptadec-8-enyl-1,3-oxazolin, dem 2-Undecyl-4-ethyl-4-hydroxymethyl-1,3-oxazolin und dem 2-Undecyl-4,4-dimethyl-1,3-oxazolin und
deren Gemischen.

8. Zusammensetzung nach einem der Ansprüche 4 bis 7, **dadurch gekennzeichnet, dass** sie für eine Verabreichung auf topischem oder oralem Weg formuliert ist.

9. Zusammensetzung nach einem der Ansprüche 4 bis 8 oder Extrakt nach einem der Ansprüche 1 bis 3 für ihre/seine Verwendung zur Vorbeugung und/oder Behandlung von Störungen oder Erkrankungen der Haut und/oder der Schleimhäute und/oder der Hautanhangsgebilde.

10. Zusammensetzung nach einem der Ansprüche 4 bis 8 oder Extrakt nach einem der Ansprüche 1 bis 3 für ihre/seine Verwendung zur Vorbeugung und/oder Behandlung von Gefäßstörungen und genauer zur Stärkung der Wände und des Gefäßtonus.

11. Zusammensetzung nach einem der Ansprüche 4 bis 8 oder Extrakt nach einem der Ansprüche 1 bis 3 für ihre/seine Verwendung zur Vorbeugung und/oder Behandlung von allergischen, entzündlichen, Reizreaktionen oder Erkrankungen oder Störungen der Barriere oder der Homöostase der unreifen, normalen oder reifen/alten Haut, Hautanhangsgebilde und/oder Schleimhäute.

12. Zusammensetzung nach einem der Ansprüche 4 bis 8 oder Extrakt nach einem der Ansprüche 1 bis 3 für ihre/seine Verwendung zur Vorbeugung und/oder Behandlung von Beeinträchtigungen des adipösen Gewebes und genauer zur Behandlung der Regulierungen des adipösen Gewebes oder zur Vorbeugung und/oder Behandlung der Störungen des Blutkreislaufs und Stärkung des Gefäßtonus.

13. Kosmetisches Pflegeverfahren der Haut und/oder der Hautanhangsgebilde und/oder der Schleimhäute zum Verbessern ihres Zustands und/oder ihres Aussehens, in vorteilhafter Weise zum Verbessern der Straffheit, der Elastizität oder der Spannkraft der Haut, und zum Bekämpfen der Bildung von adipösem Gewebe und der Haut mit Cellulite oder auch zum Vorbeugen und/oder Behandeln von Beeinträchtigungen der Dermis oder auch zum Vorbeugen und/oder Behandeln der Alterung, umfassend die Verabreichung einer kosmetischen Zusammensetzung nach einem der Ansprüche 4 bis 8 oder eines Extrakts nach einem der Ansprüche 1 bis 3.

## Claims

1. Extract of aerial parts of Maca containing at least 5% by weight of polyphenols, expressed in gallic acid equivalents, with respect to the total weight of the dry extract, said polyphenols being flavonoids having a flavonoid content greater than 2%, expressed in rutin equivalents, with respect to the total weight of the dry extract, said extract being obtainable by solid-liquid extraction of the aerial parts of Maca in binary mixtures of water/glycerol and/or water/glycol, in a proportion between 30 and 90% of glycerol and/or glycol in water.

2. Extract according to claim 1, **characterised in that** the flavonoids contain quercetin, kaempferol, derivatives thereof, or mixtures thereof, advantageously at a concentration of at least 30%, typically at least 50%, by weight, expressed in gallic acid equivalents, with respect to the total weight of the flavonoids.

3. Extract according to claims 1 or 2, **characterised in that** aerial parts are Maca leaves.

4. Composition comprising as an active compound an extract according to any of the above claims and advantageously a suitable excipient, **characterised in that** it is a cosmetic, pharmaceutical, dermatological or nutraceutical composition.

5. Composition according to claim 4, further comprising at least one further active compound in addition to the extract of aerial parts of Maca particularly chosen from the group consisting of emollients, moisturising agents, keratoregulators, keratolytics, skin barrier healing and/or remodelling agents, sebo-regulating agents, anti-irritant and/or anti-inflammatory and/or soothing agents, anti-oxidant agents, anti-ageing agents, depigmenting or hypopigmenting agents, pigmenting agents, lipolytic agents or lipogenesis inhibitors or anti-cellulite or slimming agents, mineral or organic sun filters and screens, antifungal compounds, preservatives, antibacterial agents, pre and probiotics, antibiotics, and immunomodulators.

6. Composition according to claim 5, **characterised in that** the further active substance is chosen from:
• skin barrier healing and/or remodelling agents, preferably panthenol, arabinogalactan and zinc oxide,
• sebo-regulating agents, preferably chosen from the group consisting of 5-alpha reductase inhibitors, zinc derivatives, spironolactone, and linoleic acid,
• anti-inflammatory and/or anti-irritant and/or soothing agents, preferably arabinogalactan,
• hypopigmenting or depigmenting agents, preferably N-undecylenoyl-L-phenylalanine,
• mineral or organic sun screens or filters, preferably UVB and/or UVA sun filters and screens, and
• preservatives preferably chosen from capryloyl glycine, glyceryl caprylate, hexanediol, sodium levulinate, zinc and copper derivatives.

7. Composition according to any one of claims 5 to 6, further comprising at least one further active substance chosen from the group consisting of:
• plant oils, preferably soybean oil, rapeseed oil, avocado oil, lupin oil and advantageously sweet white lupin oil, or a mixture of these oils.
• the oleodistillates or concentrates of plant or animal oils, preferably sunflower, avocado, rapeseed, corn and palm oil and advantageously concentrated in unsaponifiables,
• plant or plant oil unsaponifiables, preferably avocado unsaponifiables, soybean unsaponifiables or mixtures thereof, advantageously avocado furans, and particularly a mixture of furan avocado unsaponifiables and soybean unsaponifiables in a respective ratio of approximately 1:3-2:3, sterol unsaponifiables, plant sterols, sterol esters and vitamin derivatives,
• plant peptides or amino acid complexes, preferably avocado peptides, lupin peptides, quinoa peptides, Maca peptides, optionally fermented soybean peptides, rice peptides,
• plant sugars, preferably avocado sugars,
• butyl avocadate,
• extracts rich in polyphenols, preferably avocado extracts and extracts of aerial parts of *Gynandropsis gynandra*,
• lupeol,
• lupin total extract,
• Cupuaçu butter,
• an *Acacia macrostachya* seed extract, *Schizandra sphenanthera* seed extract and *Vigna unguiculata* seed extract,
• oxazolines, preferably 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-methyl-2-heptadecyl-1,3-oxazoline, (E)-4-hydroxymethyl-4-methyl-2-heptadec-8-enyl-1,3-oxazoline, 2-undecyl-4-ethyl-4-hydroxymethyl-1,3-oxazoline and 2-undecyl-4,4-dimethyl-1,3-oxazoline, and
mixtures thereof.

8. Composition according to any one of claims 4 to 8, **characterised in that** it is formulated for topical or oral administration.

9. Composition according to any one of claims 4 to 8 or extract as defined in any one of claims 1 to 3 for use for preventing and/or treating disorders or pathologies of the skin and/or mucous membranes and/or superficial body growths.

10. Composition according to any one of claims 4 to 8 or extract as defined in any one of claims 1 to 3 for use for preventing and/or treating vascular disorders, and more particularly for strengthening vessel walls and tonicity.

11. Composition according to any one of claims 4 to 8 or extract as defined in any one of claims 1 to 3 for use for preventing and/or treating allergic, inflammatory, irritant reactions or pathologies, or barrier or homeostasis disorders of immature, normal or mature/aged skin, superficial body growths and/or mucous membranes.

12. Composition according to any one of claims 4 to 8 or extract as defined in any one of claims 1 to 3 for use for preventing and/or treating adipose tissue alterations, and more particularly treating adipose tissue regulations, or for preventing and/or treating blood circulation disorders and strengthening vessel tonicity.

13. Cosmetic care process for the skin and/or superficial body growths and/or mucous membranes, with a view to improving the condition and/or appearance thereof, advantageously with a view to enhancing the firmness, elasticity or tonicity of the skin, and for combating the accumulation of adipose tissue and skin with cellulite, or for preventing and/or treating dermal tissue alterations, or for preventing and/or treating ageing, comprising the administration of a cosmetic composition as defined according to any one of claims 4 to 8 or an extract as defined according to any one of claims 1 to 3.
